Europäisches Patentamt

European Patent Office

Office européen des brevets

(11) Publication number: **0 221 615 B1**

# EUROPEAN PATENT SPECIFICATION

(45) Date of publication of patent specification: **12.06.91**

(51) Int. Cl.5: **B01J 37/00, B01J 23/50, B01J 35/02, B01J 37/08, B01J 37/06, C07D 301/10, C07C 45/38**

(21) Application number: **86201905.6**

(22) Date of filing: **31.10.86**

(54) Process for preparing a silver catalyst and use of same.

(30) Priority: **01.11.85 NL 8502993**

(43) Date of publication of application:
**13.05.87 Bulletin 87/20**

(45) Publication of the grant of the patent:
**12.06.91 Bulletin 91/24**

(84) Designated Contracting States:
**BE DE ES FR GB IT NL SE**

(56) References cited:
**EP-A- 0 098 764**
**FR-A- 1 137 624**

**CARBON, vol. 15, no. 4, 1977, pages 233-237, Pergamon Press, GB; R.T.K. BAKER et al.: "The behavior of silver particles supported on graphite in various gaseous environments"**

(73) Proprietor: **THE DOW CHEMICAL COMPANY**
**2030 Dow Center Abbott Road P.O. Box 1967**
**Midland Michigan 48640-1967(US)**

(72) Inventor: **Geus, John Wilhelm**
**Gezichtslaan 100**
**Nl-3723 GJ Bilthoven(NL)**
Inventor: **Meima, Garmt Ricardo**
**Aalberselaan 4c**
**Nl-3818 XM Amersfoort(NL)**
Inventor: **Bongaarts, Jacobus Elisabeth**
**Admiraalstraat 196**
**B-9120 Destelbergen(BE)**

(74) Representative: **Smulders, Theodorus A.H.J., Ir. et al**
**Vereenigde Octrooibureaux Nieuwe Parklaan 107**
**NL-2587 BP 's-Gravenhage(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention relates to a process for preparing a silver catalyst, in which the silver is applied to a thermostable inert carrier material.

Silver catalysts are used in particular in selective oxidations of organic chemical compounds. Processes in which chemical compounds are prepared on a large scale by means of silver catalysts are the oxidation of ethylene to ethylene oxide and the oxidation of methanol to formaldehyde. In addition, silver catalysts are used in the (liquid-phase) oxidation of (unsaturated) aldehydes to the corresponding carboxylic acids, such as, for example, the oxidation of acrolein or methacrolein to acrylic acid and methacrylic acid, respectively.

A major drawback of the present silver catalysts is their relatively low activity and stability. The low stability gives the most difficulties from a technical point of view. As a result, the activity of the catalyst continuously decreases in use. By increasing the temperature of the reactor, the conversion can be kept at its level, albeit at the expense of a loss in selectivity. At a given moment, however, activity has declined to the extent that the consequential increase in temperature leads to an unacceptable decrease in selectivity. The reactor must then be taken offstream so that the catalyst may be replaced by a fresh catalyst. As the replacement of the catalyst is time-consuming this leads to a considerable loss in production. Consequently, it is highly desirable to have a catalyst which, owing to better stability, permits longer service periods.

The relatively low activity of silver catalysts according to the state of the prior art is also a drawback. The low activity is caused by the catalysts containing relatively large silver particles. As a consequence, relatively much silver is needed in order to realize the silver area in the reactor necessary for the technically required conversion. The result is that the investment involved in the silver catalyst is high. The availability of catalysts with a larger thermostable silver area per unit weight of silver would render it possible to considerably decrease investments.

In FR-A 1,137,624 a silver catalyst is disclosed, comprising finely divided silver particles supported on $\alpha$-Al$_2$O$_3$ having a rugged surface.

It is accordingly an object of the present invention to provide a process for preparing a silver catalyst with a higher thermostability and a larger silver area per unit of weight of silver, which comprises depositing silver particles on an $\alpha$-aluminum oxide carrier material having steps or a stepped structure.

It has been found that silver catalysts in which the silver is applied to a carrier material of which at least 1% and preferably at least 5% of the carrier area is provided with steps or has a stepped structure, said steps or stepped structure being obtained by heating the $\alpha$-aluminum oxide carrier material with an acid prior to loading with silver particles, exhibit a surprisingly higher thermostability than the conventional silver catalysts. As small silver particles are stabilized by the steps on the carrier, the silver area and hence the activity per unit weight of silver is also larger than in silver catalysts of the prior art. It has further been found that the surprising improvement of the silver catalysts obtained according to the invention already occurs when the steps on the carrier material are just a few atomic layers high. In fact, even then, the mobility of silver particles of the carrier surface is effectively prevented.

Although there is not certainty about this, the available data suggest that the mobility of the silver particles deposited on a carrier as such leads to a decrease of the silver area. The movement of the silver particles over the carrier surface is analogous to the Brown movement which colloidal particles perform in a liquid. When thus moving silver particles come into contact with each other, they rapidly coalesce to form a larger silver particle with a smaller area than that of the original silver particles. The rapid coalescence of touching solid noble metal particles, which resembles the coalescence of liquid droplets, has been extensively observed and studied in the electron microscope with small gold particles (D.W. Pashley, Advances in Physics, Vol. 14, (1965), 327-416). For small silver particles such a coalescence like liquid droplets has also been described in the literature (R.T.K. Baker and P. Skiba Jr., Carbon 15 (1977) 233-237). The rapid coalescence of metal particles, which are solid and not liquid, is caused by the large mobility of metal atoms over metal surfaces. The driving force for the coalescence is the reduction in area and the area energy inherent therein.

In the literature, another explanation is given for the sintering of metal particles applied to inert, mostly oxidic carriers.

In it, it is supposed that atomic or molecular units of the metal particles dissociate to the surface of the carrier or even to the gaseous phase. As the dissociation of these units from small particles proceeds more readily than that from larger particles, a higher concentration of atomic or molecular units is formed around smaller particles than around larger particles. This leads to a concentration gradient, which results in migration of material from smaller to larger metal particles. In this way the smaller particles disappear and in the end only a small number of large particles remain. This has often been observed especially with particles suspended in a liquid phase. The effect is known by the name of "Ostwald ripening". The sublimation energy of silver is relatively high. Therefore, the dissociation of silver atoms is improbable at the temperature at which silver catalysts are technically used.

It is noted that Masson et al. (A. Masson, Y.Y. Metois, R. Kern, Surface science, Vol. 27, 1971, 463) have studied the movement of silver particles over a rocksalt surface, when it was found that a silver particle cannot migrate over an atomic step on the rocksalt surface. The publication in question does not, however, relate to the preparation and use of supported (silver) metal catalysts and the problem inherent therein.

According to the invention the silver catalysts can be prepared by pre-treating the α-aluminum oxide carrier with an acid. It has been found that by treating α-aluminum oxide with acid, originally rounded corners and edges of the aluminum oxide particles acquire a structure in which terraces and steps occur. A study in the scanning electron microscope has shown this. The treatment with an acid can be effected with various acids, such as hydrochloric acid, nitric acid, phosphoric acid, sulfuric acid and organic acids, such as oxalic acid, with a pK value of no more than 3. The concentration of the acid is preferably taken between 0.4N and 0.001N. In connection with the use as a catalyst and with the subsequent application of silver to the pretreated carrier, the acid-treated carrier will have to be thoroughly washed in most cases to remove all residues of the acid used as completely as possible.

The treatment with acid is advantageously carried out by suspending the carrier in an aqueous solution of the acid. The process is not limited to aqueous solutions, but other solvents may be used. Aqueous solutions, however, are preferred.

Loading a carrier having the desired stepped structure in its surface can be effected in various ways well-known to those skilled in the art. One suitable possibility is for the carrier to be impregnated with a solution of a silver compound, followed by removing the solvent by evaporation. It is also possible to start from a suspension of carrier in a solution of silver compound, and to reduce the dissolved silver with a compound such as formaldehyde or glucose to form metallic silver. The metallic silver is then deposited on the carrier.

The silver catalysts produced in accordance with the invention are distinguished by a high thermal stability. This renders these catalysts highly suitable for the selective oxidation of ethylene to ethylene oxide. Moreover, the catalysts exhibit a high selectivity for the formation of ethylene oxide, especially after a thermal pre-treatment at high temperature.

The invention is illustrated in and by the following examples.

## EXAMPLE I

The example demonstrates the decrease in mobility of silver particles on a stepped surface of an aluminum oxide carrier.

First silver particles were applied to an α-aluminum oxide carrier whose surface hardly contained steps. Subsequently, another portion of the same carrier material was treated with hydrochloric acid; the pH of the hydrochloric acid was 0.5 and the carrier was suspended in this acid for 17 hours. As will be explained hereinafter, this treatment so attacks the carrier surface at the rounded corners and edges as to form steps there. Finally, silver particles were applied to the pre-treated and carefully washed carrier in a manner identical to the non-pre-treated carrier. The silver particles were obtained by the reduction of silver ions with formaldehyde in the presence of the suspended carrier.

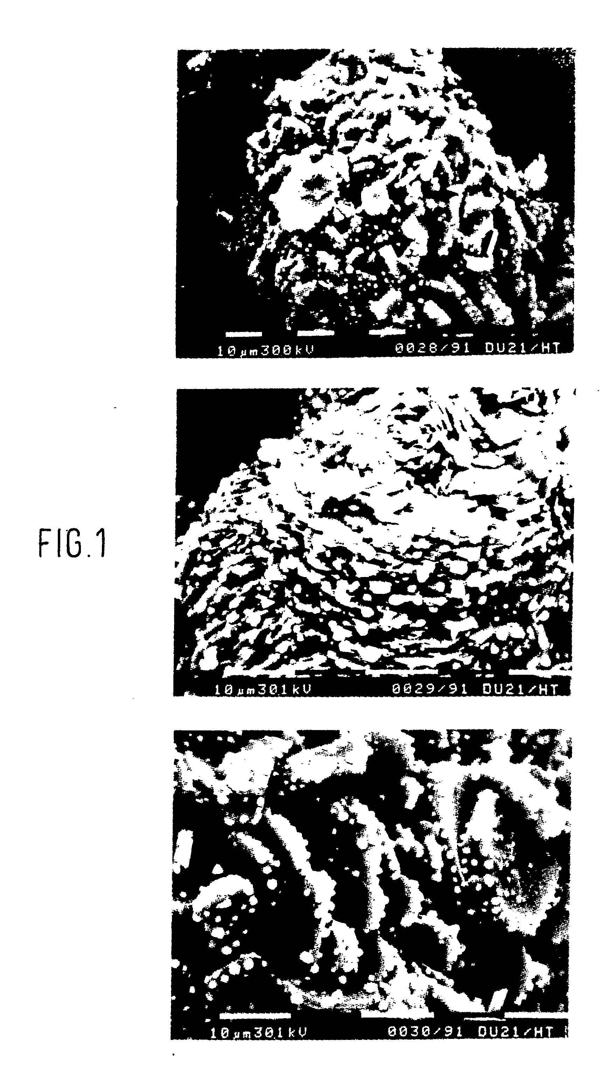

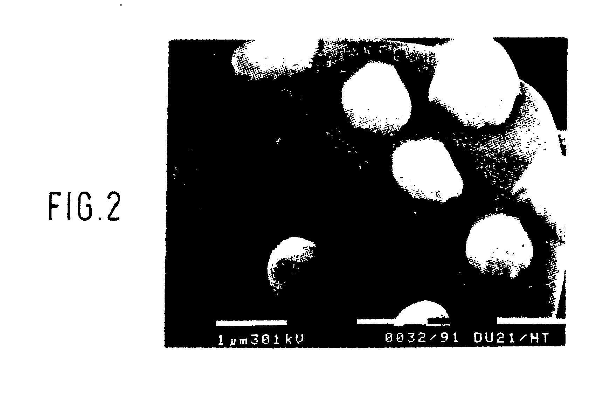

When the two catalysts were viewed in the scanning electron microscope a homogeneous distribution of the silver throughout the entire carrier material was shown. Fig. 1 shows representative pictures. Fig. 2 shows both catalysts after calcination in air at a temperature of 850°C for 19 hours. It is clearly seen that, whereas both laden carriers exhibit the same uniform distribution of silver particles over the carrier surface before the thermal treatment, this is quite different after the thermal treatment: In the carrier pre-treated with acid, after the thermal treatment the silver particles are especially present on those parts of the surface of the carrier where steps are present. In the carrier not pre-treated with acid, where steps hardly, if at all, occur in the surface, the thermal treatment leads to excessive sintering of the silver. It has been found that a fraction of 1% of the carrier surface covered with steps is sufficient to provide an appreciable improvement in thermal stability. Preferably, this fraction is 5% or more.

## EXAMPLE II

As carrier material was used α-aluminum oxide (purum) of Fluka A.G. (CH-9470 Buchs). This material has a specific area of 0.8 $m^2$/g and only possesses macropores 28.0 g of this material was suspended in an aqueous solution of hydrochloric acid with a pH of about 0.5, and kept in the solution for about 20 hours. The carrier was then filtered and thoroughly washed with deionised water until no chlorine ions could be demonstrated in the eluent. The carrier was subsequently dried at 120°C for about 2 hours.

The carrier thus pre-treated was suspended in an oxygen-free ammoniacal silver solution with a pH of about 10.5. This solution had been prepared by dissolving 3.8 g silver nitrate in 1500 ml water and then, with stirring, adding a little concentrated ammonia (25% by weight). The amount of ammonia was just sufficient to form the ammoniacal silver complex. After stabilization of the suspension for 1 hour, 20 ml concentrated formaldehyde (35% by weight) was slowly injected into the suspension with vigorous stirring. The reduction of the silver ions proceeded virtually instantaneously. After completion, the catalyst was filtered off and dried at 120°C for about 20 hours. The catalyst thus prepared contained 7.9% by weight of silver.

The activity of the dried silver-laden α-aluminum oxide powder for the oxidation of ethylene was investigated. For this purpose, 4.3 g of the powder was pressed to form pellets having sizes of 1-2 mm. The catalyst was introduced into a tubular reactor 20 cm long and 8.4 mm in diameter.

A mixture of 5% by volume of carbon dioxide, 18% by volume of ethylene, 8% by volume of oxygen and balance nitrogen was passed over the catalyst at a pressure of 4 ats. 100 ml (stp) of this gas mixture was passed over the catalyst per minute. 1 ppm of ethylene dichloride had been added to the gas mixture as a moderator. No alkali metal, or alkaline earth metal promotor had been added to the catalyst.

At a temperature of 250°C, the selectivity for the conversion of ethylene into ethylene oxide was about 80% at an ethylene conversion of about 5%.

To determine the thermal stability of the catalyst, another quantity of the powdered silver-laden carrier was kept at 850°C for 19 hours. This temperature is considerably higher than that at which silver catalysts are normally used. Therefore, an excellent impression of the thermal stability of the silver catalyst according to the invention can be obtained in relativelt short period of time, in this case 19 hours.

4.0 g of the powder thus thermally treated was pressed to form pellets (sizes 1 mm - 2 mm) and introduced into the above described reactor. A mixture of 5% by volume of carbon dioxide, 18% by volume of ethylene, 8% by volume of oxygen and balance nitrogen was passed over the catalyst at a pressure of 4 ats. 1 ppm of ethylene dichloride had been added to the gas mixture at a moderator. Again, no alkali metal or alkaline earth metal promotor had been added.

Under these reaction conditions, at a reactor temperature of 280°C, a selectivity for the conversion of ethylene into ethylene oxide of more than 75% was measured at a conversion of about 8%.

EXAMPLE III (Comparative example)

In the same way as described in Example II, a catalyst was prepared, but now the carrier was not previously treated with an acid.

The catalyst was prepared, starting from 27.8 g carrier material and 3.82 g silver nitrate. Ultimately the catalyst contained 8.0% by weight of silver. The reactor was loaded in the same manner as described above, and the reaction mixture was also identical to that described above. At a temperature of 220°C, the selectivity for the conversion of ethylene into ethylene oxide of the (fresh) catalyst not thermally treated was about 80% at an ethylene conversion of about 4%. After a thermal treatment at 850°C in air for 19 hours, at a reactor temperature of 280°C, a selectivity of about 65% was measured with an ethylene conversion of about 4%.

The above examples show that, by providing steps on the α-aluminum surface, the thermal stability of the silver particles has been increased. Although the differences between the two "fresh" catalysts are not large, the superiority of the catalyst according to the invention if apparent after the thermal pre-treatment at 850°C. Both a higher activity and a higher selectivity was observed with the catalyst prepared according to the invention.

**Claims**

1. A process for preparing a silver catalyst, which comprises depositing silver particles on an α-aluminum oxide carrier material having steps or a stepped structure, characterized by using a carrier material in which at least 1% of the surface is provided with steps or has a stepped structure, said steps or stepped structure being obtained by heating the α-aluminum oxide carrier material with an acid prior to loading with silver particles.

2. A process as claimed in claim 1, characterized by using a carrier material in which at least 5% of the surface is provided with steps or has a stepped structure.

3. A process as claimed in claim 1 or 2, characterized by heating the carrier material at a temperature higher than 1000°C prior to loading with silver particles.

4. A process as claimed in claim 1-3, characterized in that the carrier is washed with water after the treatment with acid.

5. A process as claimed in claim 4, characterized in that the carrier is washed until no acid residues from the treatment with acid can be demonstrated in the washing water.

6. A process as claimed in claims 1-5, characterized by using an acid having a pKa $\leq$ 3.

7. A process as claimed in claims 1-6, characterized in that the acid is selected from the group consisting of HCl, $HNO_3$, $H_3PO_4$ and $H_2SO_4$, and oxalic acid.

8. A process as claimed in claims 1-7, characterized in that the carrier is treated with a solution of an acid having a pH < 4.

9. A process for carrying out catalytic selective oxidation reactions, in particular the oxidation of ethylene to ethylene oxide or the oxidation of methanol to formaldehyde, characterized by using a catalyst produced by the process as claimed in any of claims 1-8.

**Revendications**

1. Procédé de fabrication d'un catalyseur à base d'argent, consistant à déposer des particules d'argent sur un matériau de support en oxyde d'aluminium $\alpha$, comportant des gradins ou présentant une structure en gradins, caractérisé en ce que l'on utilise un matériau de support dans lequel au moins 1 % de la surface est muni de gradins ou présente une structure en gradins, lesdits gradins ou ladite structure en gradins étant obtenus par chauffage du matériau de support en oxyde d'aluminium $\alpha$ avec un acide, avant le dépôt des particules d'argent.

2. Procédé selon la revendication 1, caractérisé en ce que l'on utilise un matériau de support dans lequel au moins 5 % de la surface sont munis de gradins ou présentent une structure en gradins.

3. Procédé selon la revendication 1 ou 2, caractérisé en ce que l'on chauffe le matériau de support à une température supérieure à 1000° C avant le dépôt des particules d'argent.

4. Procédé selon les revendications 1-3, caractérisé en ce que le support est lavé à l'eau après le traitement avec l'acide.

5. Procédé selon la revendication 4, caractérisé en ce que le support est lavé jusqu'à ce qu'aucun résidu acide provenant du traitement avec l'acide ne puisse plus être décelé dans l'eau de lavage.

6. Procédé selon les revendications 1-5, caractérisé en ce que l'on utilise un acide ayant un pKa $\leq$ 3.

7. Procédé selon les revendications 1-6, caractérisé en ce que l'acide est choisi dans le groupe constitué par HCl, $HNO_3$, $H_3PO_4$ et $H_2SO_4$, et l'acide oxalique.

8. Procédé selon les revendications 1-7, caractérisé en ce que le support est traité avec une solution d'acide ayant un pH < 4.

9. Procédé pour la réalisation de réactions catalytiques sélectives d'oxydation, en particulier l'oxydation de l'éthylène en oxyde d'éthylène ou l'oxydation du méthanol en formaldéhyde, caractérisé en ce que l'on utilise un catalyseur produit par le procédé selon l'une quelconque des revendications 1-8.

**Ansprüche**

1. Verfahren zur Herstellung eines Silberkatalysators durch Ablagerung von Silberteilchen auf einem $\alpha$-Aluminiumoxid-Trägermaterial mit Stufen oder einer stufenförmigen Struktur, dadurch gekennzeichnet, dar man ein Trägermaterial verwendet, in dem mindestens 1 % der Oberfläche mit Stufen versehen ist oder eine stufenförmige Struktur besitzt, wobei die Stufen oder die stufenförmige Struktur durch Erhitzen des $\alpha$-Aluminiumoxid-Trägermaterials mit einer Säure vor der Beladung mit den Silberteilchen erhalten wird.

2. Verfahren nach Anspruch 1, dadurch gekennzeichnet, dar man ein Trägermaterial verwendet, in dem mindestens 5 % der Oberfläche mit Stufen versehen ist oder eine stufenförmige Struktur besitzt.

3. Verfahren nach Anspruch 1 oder 2, dadurch gekennzeichnet, daß man das Trägermaterial vor dem Beladen mit Silberteilchen auf eine Temperatur von höher 1000° C erhitzt.

4. Verfahren nach Anspruch 1 bis 3, dadurch gekennzeichnet, daß der Träger nach der Behandlung mit Säure mit Wasser gewaschen wird.

5. Verfahren nach Anspruch 4, dadurch gekennzeichnet, daß der Träger gewaschen wird, bis keine Säurereste aus der Behandlung mit Säure in dem Wasser nachgewiesen werden können.

6. Verfahren nach den Ansprüchen 1 bis 5, dadurch gekennzeichnet, daß man eine Säure verwendet, die einen pka-Wert $\leq$ 3 besitzt.

7. Verfahren nach den Ansprüchen 1 bis 6, dadurch gekennzeichnet, daß die Säure ausgewählt ist aus der Gruppe bestehend aus HCl, $HNO_3$, $H_3PO_4$ und $H_2SO_4$, und Oxalsäure.

8. Verfahren nach den Ansprüchen 1 bis 7, dadurch gekennzeichnet, dar man den Träger mit einer Säurelösung mit einem pH-Wert < 4 behandelt.

9. Verfahren zur Durchführung katalytisch selektiver Oxidationsreaktionen, insbesondere der Oxidation von Ethylen zu Ethylenoxid oder der Oxidation von Methanol zu Formaldehyd; dadurch gekennzeichnet, daß man einen Katalysator verwendet, der nach dem Verfahren nach einem der Ansprüche 1 bis 8 hergestellt wurde.

FIG.1

FIG.2